# FASCICULE DE BREVET EUROPEEN

(11) **EP 0 254 655 B1**
(45) Date de publication et mention de la délivrance du brevet: **21.04.1993**
(21) Numéro de dépôt: 87440040.1
(22) Date de dépôt: 24.06.1987
(51) Int. Cl.: C07J 41/00, C07J 43/00, A61K 31/57, A61K 31/58

(54) **Composés de synthèse associative d'acides aminés soufrés ou non soufrés avec des dérivés du pregnane, leur préparation et leur utilisation**
Syntheseprodukte aus Schwefel oder nichtschwefelhaltigen Aminosäuren mit Pregnanderivaten, ihre Herstellung und ihre Anwendung
Associated synthesis compounds from sulfurized or non-sulfurized amino acids with pregnane derivatives, their preparation and their use

(30) Priorité: 24.06.1986 FR 8609246
(43) Date de publication de la demande: 27.01.1988
(73) Titulaire: Université Louis Pasteur de Strasbourg Etablissement public à caractère scientifique et culturel, F-67000 Strasbourg (FR)
(72) Inventeur: Milioni, Catherine, G-16341 Ilioupoli (GR); Efthyimiopoulos, Constantin, G-16341 Ilioupoli (GR); Koch, Bernard, F-67100 Strasbourg (FR); Jung, Louis, F-67200 Strasbourg (FR); Jung, Jean, F-67200 Strasbourg (FR)
(74) Mandataire: Nuss, Pierre

(56) Documents cités:
- FR-A- 1 272 464
- FR-A- 2 077 750
- FR-A- 2 280 383
- FR-A- 2 459 249
- GB-A- 962 797
- JOURNAL OF PHARMACEUTICAL SCIENCES, vol. 74, no. 1, janvier 1985, pages 87-89, American Pharmaceutical Association, Washington, D.C., US; K. JOHNSON et al.: "Solution kinetics of a water-soluble hydrocortisone prodrug: hydrocortisone-21-lysinate"

## Description

L'invention a pour objet des nouveaux composés de synthèse associative d'acides aminés soufrés ou non soufrés avec des dérivés du Δ-4 pregnène 3,20-dione ou avec des dérivés du Δ-1,4 prégnadiène 3,20-dione, ainsi que leurs sels d'addition avec les acides minéraux ou organiques en présence du groupement aminé ou leurs sels minéraux en présence du groupement carboxylique, leurs procédés de préparation, leur activité pharmacologique et les compositions pharmaceutiques les renfermant.

Actuellement, les stéroïdes anti-inflammatoires présentant une hydrosolubilité ont déjà été décrits, en particulier en vue de leur utilisation par voie injectable. A cet effet, la synthèse des esters en position 21 et/ou 17 de stéroïdes avec des acides polybasiques ou des acides aminés est déjà décrite. On connait d'ailleurs de GB-A- 962 797 un ester de la glycine en association avec la fonction amine libre.

D'autre part, des stéroïdes anti-inflammatoires obtenus par synthèse associative entre des acides aminés et des stéroïdes présentant un groupement ester en 21 et soufrés au niveau de la chaîne latérale, mais ne présentant pas de groupement aminé libre salifiable ont également déjà été décrits. De tels stéroïdes sont connus de FR-A-2 077 750 qui a pour objet des stéroïdes anti-inflammatoires à usage topique présentant un groupement soufré et dont la fonction amine est protégée.

La présente invention concerne des composés anti-inflammatoires stéroïdiens résultant d'une synthèse associative entre le stéroïde et les acides aminés et présentant un effet réservoir associé à un tropisme cutané où un groupement hydrosoluble sous forme de sel minéral ou organique est favorable.

Pour obtenir cette double activité conjointe, les nouvelles structures revendiquées présentent, et répondent aux trois formules générales I, II et III :
. Soit une liaison ester en 21 mais comprenant une structure amino acide soufrée au groupement amine libre (formule générale I),
. Soit un groupement aminé en 21 d'origine amino acide soufré ou non (formule II),
. Soit un groupement soufré en 21 et une structure d'amino acide terminale (formule III).

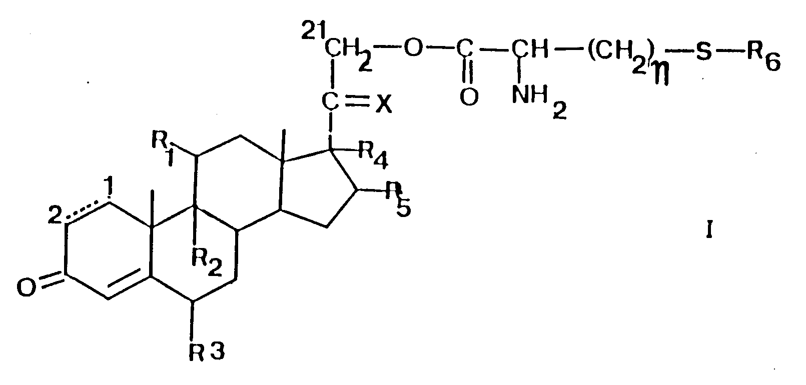

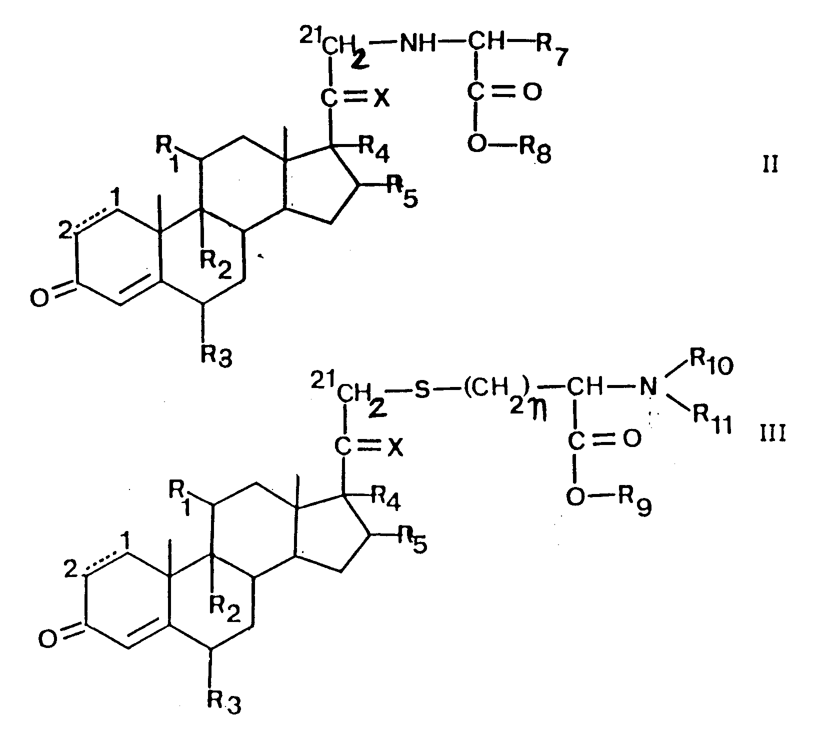

dans lesquelles:
- pour les trois formules générales I, II et III
   la liaison C₁-C₂ peut être une liaison saturée(dérivés de l'hydrocortisone p.ex). ou peut être une liaison éthylénique (dérivés de la dexaméthesone p.ex);
   R₁ représente un atome d'oxygène, ou de soufre, ou de chlore, ou encore un hydroxyle;
   R₂ et/ou R₃ représentent un atome d'hydrogène, ou de fluor, ou de chlore, ou encore un groupement méthyle;
   R₄ et/ou R₅ représentent un hydrogène, ou un hydroxyle, ou un méthyle, ou un éthyle ; R₅ pouvant aussi être un méthylène;
   R₄ et R₅ peuvent former un hétérocycle du type-O-CH(Y)-O- ou -O-C(Y)₂-O-,
   Y étant un hydrogène ou un groupe alkyle linéaire ou ramifié ayant de préférence 1 à 4 atomes de carbone;
   X représente un oxygène,ou un soufre;
- pour la formule I
   n vaut 1 ou 2;
   R₆ peut représenter un méthyle, ou un groupement arylalkyle, ou tout autre groupement utilisé pour la protection de la fonction thiol;
   R₆ peut correspondre à l'ensemble de la structure I donnant une duplication de la formule I avec formation d'un pont disulfure;
- pour la formule II
   R₇ représente un hydrogène d'origine glycine, ou un groupe alkyle linéaire, en particulier un méthyle d'origine alanine, ou un groupe alkyle ramifié, d'origine valine, leucine, isoleucine, ou un groupe hydroxyalkyle linéaire, d'origine sérine, thréonine, ou un groupe thioalkyle d'origine méthionine, ou un groupe thiolalkyle d'origine cystéine, ou un groupe arylalkyle soit p.ex. benzyle d'origine phénylalanine,soit p.ex. hydroxybenzyle d'origine tyrosine, soit p.ex. indolylalkyle d'origine tryptophane, soit imidazolylalkyle d'origine histidine, ou un groupe amidoalkyle d'origine asparagine, glutamine, ou un groupe alkyle carboxylique sous forme libre ou salifiée par le sodium p.ex., d'origine acide aspartique, acide glutamique ou cyclisé sous forme d'acide pyroglutamique ou un groupe amino alkyle d'origine lysine, ou un groupe guanidinoalkyle d'origine arginine, ou encore un groupe dithiodiméthyle, d'origine cystine, fixant symétriquement deux glucocorticoïdes;
   le groupement NH peut former avec R₇ un hétérocycle azoté du type pyrrolidine d'origine proline;
   les différents acides aminés cités peuvent être remplacés par leurs homologues supérieurs;
   R₈ peut être un hydrogène, ou un sel correspondant du type, sodium, potassium, calcium;
   R₈ peut aussi être un groupe alkyle linéaire ou ramifié ayant de préférence 1 à 4 atomes de carbone, ou un groupe arylalkyle du type benzyle ou hétérocyclique;
- pour la formule III
   n vaut de 1 à 6, mais on préfére n = 1 d'origine cystéine, ou n = 2 d'origine homocystéine;
   R₉ peut prendre les différentes structures évoquées pour R₈;
   R₁₀ et/ou R₁₁ peuvent représenter un hydrogène, ou un groupement alkyle linéaire ou ramifié ayant de préférence 1 à 3 atomes de carbone, structure sous forme aminée ou ammonium;
   R₁₀ ou R₁₁ peuvent également avoir une structure -CO-Z-, Z étant un groupement alkyle linéaire ou ramifié, ou arylalkyle.

L'invention a aussi pour objet des procédés de préparation des dérivés, tels que définis par les formules I, II et III, ci-dessus.

Pour les dérivés de formule I, le procédé consiste aux transformations successives du stéroïde en mésylate puis en dérivé iodé, ce dernier réagissant avec la fonction carboxylique d'un acide aminé dont la fonction amine est protégée par le groupement tertio-butyloxycarbonyle. Par la suite, le groupement protecteur de la fonction amine est éliminé. Le stéroïde peut être l'hydrocortisone, ou la dexaméthasone ou tout autre stéroïde glucocorticoïde à chaîne -CO-CH₂OH, l'acide amineé est en particulier la cystine, la méthionine, ou encore la cystéine dont la fonction thiol est protégée.

Pour les dérivés de la formule II, le procédé consiste aux transformations successives du stéroïde en mésylate puis en dérivé iodé, ce dernier réagissant avec la fonction amine d'un acide aminé dont la fonction carboxylique est protégée par différents groupements dont les esters. La réaction peut être réalisée dans les mêmes conditions à partir du mésylate-21 du stéroïde. Le stéroïde peut être l'hydrocortisone ou la dexaméthasone ou tout autre stéroïde glucocorticoïde à chaîne -CO-CH₂OH, l'acide aminé peut être en particulier la cystine, la méthionine, l'acide glutamique, ou encore l'acide pyroglutamique.

Pour les dérivés de la formule III, le procédé consiste à la transformation du stéroïde en mésylate qui réagit avec un acide aminé présentant un groupement thiol SH libre, tel que par exemple, la cystéine, ou homocystéine.

L'invention a aussi pour objet la préparation des composés définis par les formules I, II et III, sous forme de sels d'addition avec les acides minéraux ou organiques en présence du groupement aminé, ou leurs sels minéraux en présence du groupement carboxylique, en particulier sous forme de carboxylates de sodium, de potassium, ou de calcium.

Les composés de formules I, II, ou III sont caractérisés en ce qu'ils représentent avantageusement l'un des composés ci-après:
- ester entre l'hydrocortisone et la cystine (formule I);
- ester entre la dexaméthasone et la cystine (formule I);
- ester entre l'hydrocortisone et la méthionine (formule I);
- ester entre la dexaméthasone et la méthionine (formule I);
- amine en C₂₁ entre l'hydrocortisone et la méthionine (formule II);
- amine en C₂₁ entre la dexaméthesone et la méthionine (formule II);
- amine en C₂₁ entre l'hydrocortisone et la cystine (formule II);
- amine en C₂₁ entre la dexaméthasone et la cystine (formule II);
- éther thiol en 21 entre l'hydrocortisone et la cystéine (formule III);
- éther thiol en 21 entre la dexaméthasone et la cystéine (formule III);
L'invention va maintenant être décrite plus en détail dans les exemples donnés ci-après à titre non limitatif, parmi lesquels:

### Exemple 1 : Synthèse de diesters de la cystine à partir de stéroïdes anti-inflammatoires. (exemple correspondant aux composés de formule générale I).

Le stéroïde est transformé en mésylate-21 par l'action de chlorure d'acide méthane sulfonique dans la pyridine. Le dérivé synthétisé a été transformé par l'action de l'iodure de sodium dans l'acétone, en dérivé iodé. Le diester a été formé par la réaction du dérivé iodé avec la cystine, dont la fonction amine a été préalablement protégée par le groupement tertio-butyloxycarbonyle, dans le DMF et en présence de triéthylamine. Le produit final au groupement amine libre a été obtenu sous forme de sel d'addition avec l'acide bromhydrique, après avoir éliminé le groupement tertio-butyloxycarbonyle par l'action d'un mélange d'acide acétique/acide bromhydrique.

Le produit final a été caractérisé sur la plaque chromatographique par la coloration spécifique (rouge sur fond jaune) des disulfures, après pulvérisation avec une solution de nitroprussiate de sodium, suivie par une pulvérisation avec une solution de cyanure de sodium. La caractérisation a été complètée par des analyses spectrales en infra-rouge et résonance magnétique nucléaire. La structure a été vérifiée par la détermination de la masse moléculaire en spectrométrie de masse.

### Exemple 2. : Synthèse de stéroïdes anti-inflammatoires aminés et soufrés (composés de formule générale II)

Le dérivé iodé du stéroïde réagit dans le DMF avec la méthionine, préalablement estérifiée, pour former le stéroïde aminé et soufré. Le composé désiré est finalement obtenu sous forme de chlorhydrate après le traitement approprié du milieu réactionnel( ac. chlorhydrique gazeux dans le méthanol, puis addition de l'éther éthylique)
La réaction peut être réalisée dans les mêmes conditions, à partir du mésylate-21 du stéroïde.

Pour les composés de formule générale III, le procédé de préparation est identique à celui des composés de formule générale II en faisant réagir la cystéine sur le mésylate-21 d'hydrocortosine ou de dexaméthasone.

Les méthodologies de préparation des différents dérivés sont les suivants:

La préparation des intermédiaires de synthèse est décrite pour la dexaméthasone qui peut être remplacée par tout stéroïde glucocorticoïde présentant en 21 une chaîne-CO- CH₂OH.
*Méthane sulfonate-21 de dexaméthasone

500mg (1.3 mmol) de dexaméthasone sont dissous dans 8 ml de pyridine anhydre et la solution est agitée à 0°C. 0,4 ml de chlorure d'acide méthane sulfonique sont ajoutés. La solution est agitée à 0°C pendant 1h. 0,2 ml de chlorure de l'acide méthane sulfonique sont ajoutés dans la solution et l'agitation est poursuivie pendant 30 min supplémentaires. Verser la solution dans l'eau froide sous forte agitation. Il se forme un précipité blanc qui est filtré et soigneusement lavé à l'eau, puis seché sur P₂₀₅ dans un dessicateur sous vide.
P.F.=246°C rendement:85 %
*Iodo-21 de dexaméthasone
520 mg (1,1 mmol) de méthane sulfonate-21 de dexaméthasone sont dissous dans 10 ml d'acétone anhydre, puis 600 mg d'iodure de sodium anhydre sont ajoutés. Le mélange est porté deux heures à reflux, puis après élimination de l'excès d'acétone, le résidu est versé dans de l'eau sous forte agitation. Il se forme un précipité jaune pâle qui est filtré et lavé à l'eau, puis séché sur P₂₀₅.
P.F.:210°C rendement:75 %
*Cystine di-(desoxy-21 dexaméthasone) ester dibromhydrate
1g (3,4 mmoles) de N,Nʹ di-t-butyloxycarbonyl cystine et 5,5 ml. de triéthylamine sont ajoutés dans 20 ml de DMF anhydre. La solution est agitée pendant quelques minutes, puis 3,01gr (6,3 mmoles) de iodo-21 de dexaméthasone sont ajoutés. Après 2 heures d' agitation à 40°C, la solution est versée dans 100 ml d'acétate d'éthyle. La phase organique est lavée succesivement à l'eau, par une solution de NaHCO₃ à 5 %, par une solution aqueuse de HCl 1 % et finalement à l'eau. La phase organique est séchée sur MgSO₄ et évaporée à sec.

Le produit a été obtenu sous forme de sel d'addition avec l'acide bromhydrique, après avoir éliminé le groupement tertio-butyloxycarbonyle par l'action d'un mélange d'acide acétique/acide bromhydrique.
P.F.: 220°C rendement:12 %(après la déprotection)
Les mêmes modes opératoires ont été suivis pour la synthèse des dérivés à partir de l'hydrocotisone, ainsi que pour les dérivés de la cystine avec d'autres groupements protecteurs de la fonction amine.
*N-(desoxy-21 hydrocortisone)-21 méthionylate de méthyle
0,45 gr (2,2 mmoles) d' ester méthylique de méthionine sous forme de chlorhydrate sont dissous dans 30 ml de DMF anhydre. 4,76 ml de triéthylamine sont ajoutés et la solution est agitée pendant quelques minutes. Après l'addition de 1 gr (2,28 mmoles) de méthane sulfonate-21 d'hydrocortisone, le mélange réactionnel est chauffé à 40°C pendant 2 heures. La solution est versée dans 150 ml d'eau froide et extraite 3 fois par 100 ml d'AcOEt. La phase organique est lavée par une solution saturée de NaCl puis séchée sur MgSO₄ et évaporée sous vide. Le résidu est dissous dans le méthanol et transformé en chlorhydrate par la réaction d'acide chlorhydrique gazeux. Après l'addition d'éther anhydre il se forme un précipité blanc qui est filtré, puis séché sur P₂₀₅.
P.F.:238°C, déc. rendement:60 %
Dans le but d'illustrer l'invention de façon non limitative, des résultats de l'étude pharmacologique qui a été menée en rapport avec ces substances sont exposés ci-après; et sont expliqués avec références aux dessins shématiques annexés dans lesquels:
- la figure 1 représente l'effet des dérivés synthètisés sur la sécrétion de l'α-MSH, induite par le CRF, chez des cellules corticotropes en culture ( courbe 1: sans CRF, courbe 2: dexaméthasone, courbe 3:hydrocortisone aminé et soufré, n=15);
- la figure 2 représente l'effet des dérivés synthètisés sur la sécrétion de l'ACTH, induite par le CRF, chez des cellules corticotropes en culture ( courbe 4: sans CRF, courbe 5: dexaméthasone, courbe 6: dexaméthasone soufrée, n=15).

On a trouvé que les composés selon l'invention peuvent être appliqués pour la fabrication d'un médicament destiné à une utilisation thérapeutique comme anti-inflammatoire, particulièrement au niveau cutané et ophtalmique.

Dans un premier temps, on effectue l'étude des effets des molécules synthétisées sur la sécrétion de l'α-MSH ou de l'ACTH, induite par le CRF, chez des cellules tumorales corticotropes AtT-20 en culture.

Le principe de ce test siège à la capacité des cellules corticotropes (dérivés d'une tumeur de l'hypophyse antérieure)de sécréter la corticotropine (ACTH), l'α-MSH (α-Mélanocyte Stimulating Hormone) et autres peptides avec une activité qui ressemble à celle des endorphines.

Cette sécrétion est stimulée par le facteur d'hypothalamus CRF (Corticotropin-Releasing Factor).

La diminution donc de la sécrétion de l'α-MSH ou de l'ACTH est indicative de l'activité glucocorticoïde des stéroïdes synthètisés. La quantité de l'α-MSH ou de l'ACTH sécrétée a été déterminée par des méthodes radioimmunologiques (RIA).

Les cellules tumorales corticotropes AtT-20 ont été incubées, dans un milieu de culture constitué de 90% DME (Dulbecco's modified Eagle's medium) et 10 % de sérum (fetal bovine serum). L'incubation est de 6 heures en présence de stéroïdes à la concentration de 10⁻⁹, 10⁻⁸ et 10⁻⁷ M.

Les cellules sont ensuite lavées et préincubées pendant 60 minutes dans le milieu sans sérum.

Après addition de CRF( 10 nM) les cellules ont été incubées pendant 90 minutes en présence des stéroïdes aux concentrations données précédement.

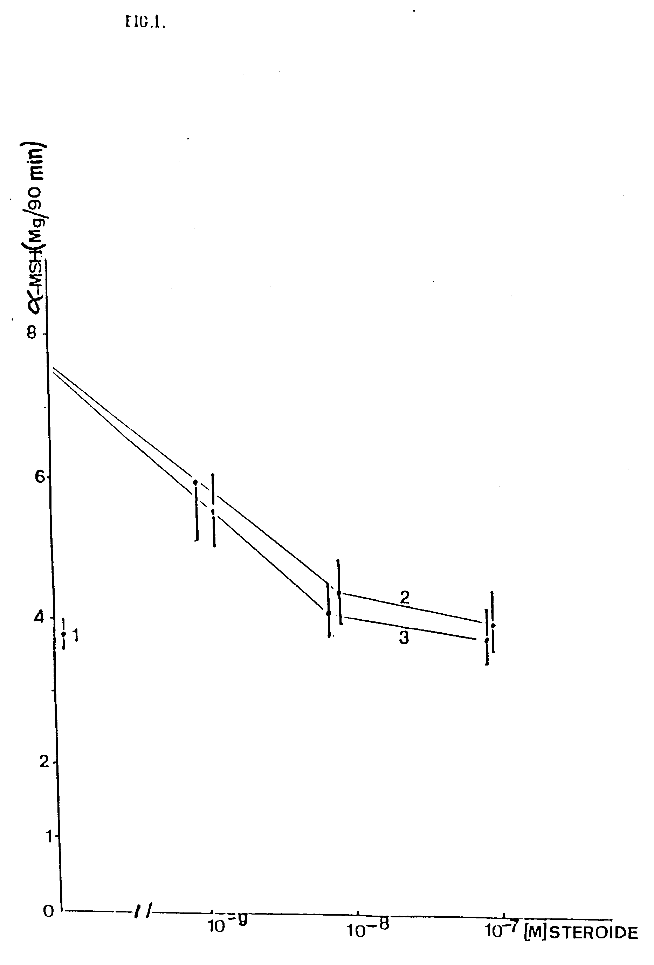

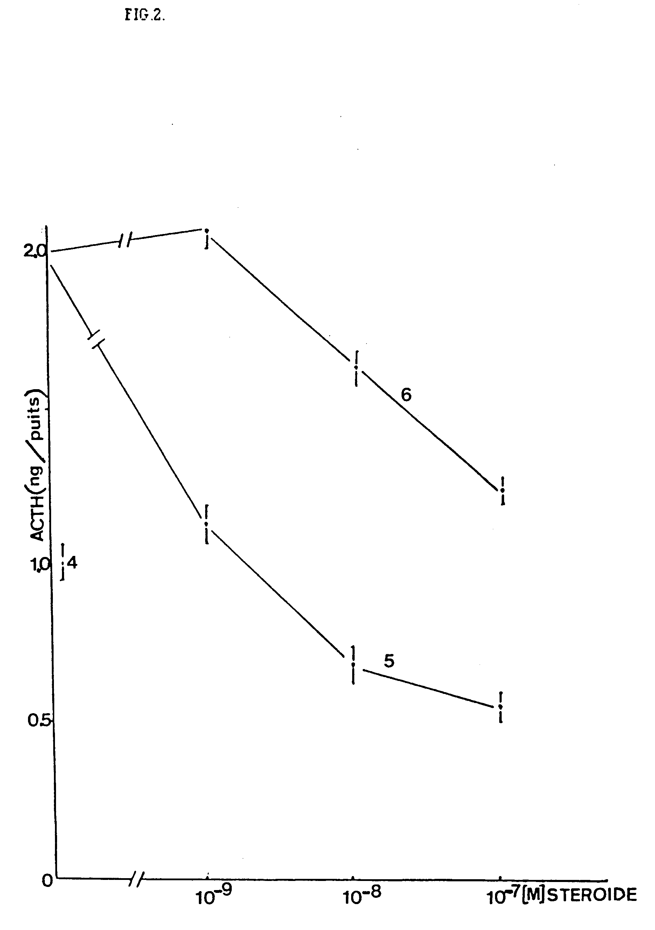

L'effet des molécules synthétisées sur la sécrétion de l'α-MSH ou de l'ACTH, induite par le CRF, chez les cellules corticotropes AtT-20 en culture, est représenté à la figure 1 et à la figure 2, respectivement.

L'incubation réalisée en absence des stéroïdes et de CRF a servi pour le control.

L'activité glucocorticoïde de l'hydrocortisone aminé et soufré ( figure 1) est équivalente à celle de dexaméthasone.

En ce qu'il concerne la dexaméthasone soufrée est envrion 10 fois moins active que la dexaméthasone (figure 2).

Les résultats montrent qu'une fonction amine ou un thioéther peuvent remplacer l'alcool primaire de la chaîne latérale du stéroïde anti-inflammatoire sans entraîner une diminution importante de l'activité glucocorticoïde.

Dans un second temps on effectue l'étude de l'activité anti-inflammatoire locale des dérivés synthétisés selon la technique de l'abcès à la carragénine.

La technique de l'abcès à la carragénine consiste à injecter par voie sous-cutanée à un rat d'une solution à 2% de carragénine, qui développe une réaction inflammatoire caractérisée par la formation d'un abcès. L'application topique de stéroïde reduit cette réponse inflammatoire qui est quantifiée par la pesée du granulôme après disection.

Les nouveaux composés décrits sont au moins aussi actifs que l'hydrocortisone ou que la dexaméthasone pris comme référence.

Des exemples de préparations pharmaceutiques incluant ces nouveaux composés de synthèse associative peuvent être présentés:

### Collyre:

Stéroïde soufré et/ou aminé 5 à 100 mg
excipients : phosphate monosodique anhydre, hydrogenophosphate de sodium, chlorure de sodium, hydroxyéthylcellulose, polysorbate 80, eau distillée.

### Crème :

Stéroïde soufré et/ou aminé 5 à 100 mg
excipients : alcool cétylstéarique, PEG 1000 monocétylether, huile de vaseline, vaseline, parahydroxybenzoate de méthyle et de propyle, acide citrique, citrate de sodium, eau distillée.

### Pommade :

Stéroïde soufré et/ou aminé 5 à 100 mg
excipients : polyethylène et huile de paraffine

### Lotion :

Stéroïde soufré et/ou aminé 5 à 100 mg
excipients : glycérine, alcool isopropylique, polyvidone, excipient, acide citrique, citrate de sodium, eau distillée.

Les stéroïdes soufrés en se fixant sur la kératine de la peau sont des principes actifs fortement fixés au niveau de l'épiderme (effet réservoir); leur libération n'est que progressive et uniforme dans le temps sous l'influence des estérases cutanées.

Les stéroïdes soufrés et aminés ont un effet réservoir en se fixant au niveau de l'épiderme et un effet d'activité augmentée.

## Revendications

1. Composés, caractérisés en ce qu'ils sont de synthèse associative d'acides aminés soufrés ou non soufrés avec des dérivés du Δ-4 pregnène 3,20-dione ou avec des dérivés du Δ-1,4 prégnadiène 3,20-dione, et en ce qu'ils répondent aux trois formules générales I, II et III: 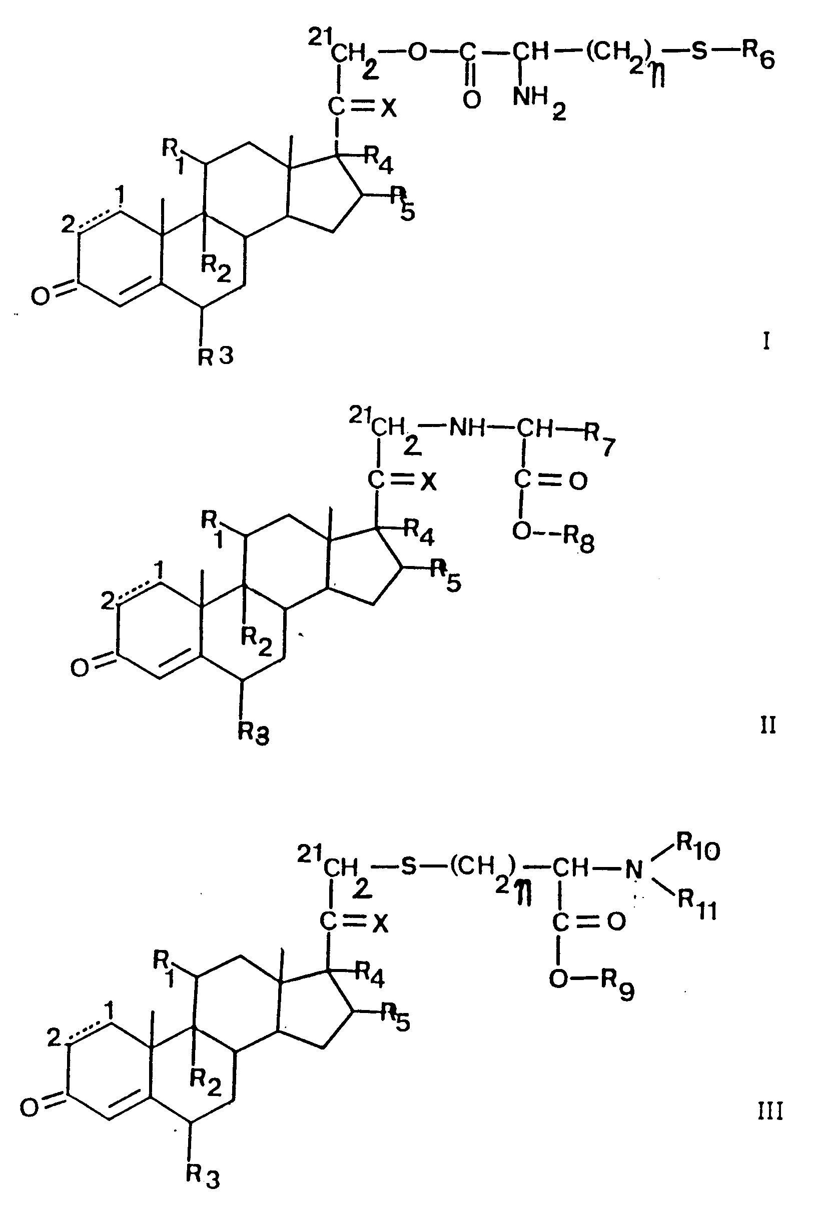 dans lesquelles:
- pour les trois formules générales I, II et III,
la liaison C₁-C₂ peut être une liaison saturée (dérivés de l'hydrocortisone p.ex). ou peut être une liaison éthylénique (dérivés de la dexaméthasone p.ex);
R₁ représente un atome d'oxygène, ou de soufre,ou de chlore,ou encore un hydroxyle;
R₂ et/ou R₃ représentent un atome d'hydrogène, ou de fluor, ou de chlore,ou encore un groupement méthyle;
R₄ et/ou R₅ représentent un hydrogène, ou un hydroxyle, ou un méthyle,ou un éthyle; R₅ pouvant aussi être un méthylène;
R₄ et R₅ peuvent former un hétérocycle du type-O-CH(Y)-O- ou -O-C(Y)₂- O-,
Y étant un hydrogène ou un groupe alkyle linéaire ou ramifié ayant de préférence 1 à 4 atomes de carbone;
X représente un oxygène, ou un soufre;
- pour la formule I
n vaut 1 ou 2;
R₆ peut représenter un méthyle, ou un groupement arylalkyle, ou tout autre groupement utilisé pour la protection de la fonction thiol;
R₆ peut correspondre à l'ensemble de la structure I donnant une duplication de la formule I avec formation d'un pont disulfure;
- pour la formule II
R₇ représente un hydrogène d'origine glycine,ou un groupe alkyle linéaire,en particulier un méthyle d'origine alanine, ou un groupe alkyle ramifié, d'origine valine, leucine, isoleucine, ou un groupe hydroxyalkyle linéaire, d'origine sérine, thréonine, ou un groupe thioalkyle d'origine méthionine, ou un groupe thiolalkyle d'origine cystéine, ou un groupe arylalkyle soit p.ex. benzyle d'origine phénylalanine, soit p.ex. hydroxybenzyle d'origine tyrosine, soit p.ex. indolylalkyle d'origine tryptophane, soit imidazolylalkyle d'origine histidine, ou un groupe amidoalkyle d'origine asparagine, glutamine, ou un groupe alkyle carboxylique sous forme libre ou salifiée par le sodium p.ex., d'origine acide aspartique, acide glutamique ou cyclisé sous forme d'acide pyroglutamique, ou un groupe amino alkyle d'origine lysine, ou un groupe guanidinoalkyle d'origine arginine, ou encore un groupe dithiodiméthyle, d'origine cystine, fixant symétriquement deux glucocorticoïdes;
le groupement NH peut former avec R₇ un hétérocycle azoté du type pyrrolidine d'origine proline;
les différents acides aminés cités peuvent être remplacés par leurs homologues supérieurs;
R₈ peut être un hydrogène, ou un sel correspondant du type, sodium, potassium, ou calcium;
R₈ peut aussi être un groupe alkyle linéaire ou ramifié ayant de préférence 1 à 4 atomes de carbone, ou un groupe arylalkyle du type benzyle ou hétérocyclique;
- pour la formule III
n vaut de 1 à 6, mais on préfére n = 1 d'origine cystéine, ou n = 2 d'origine homocystéine;
R₉ peut prendre les différentes structures évoquées pour R₈;
R₁₀ et/ou R₁₁ peuvent représenter un hydrogène, ou un groupement alkyle linéaire ou ramifié ayant de préférence 1 à 3 atomes de carbone, structure sous forme aminée ou ammonium;
R₁₀ ou R₁₁ peuvent également avoir une structure -CO-Z-, Z étant un groupement alkyle linéaire ou ramifié, ou arylalkyle.

2. Procédé de préparation des dérivés de formule I, selon la revendication 1, obtenus par transformations successives du stéroïde en mésylate puis en dérivé iodé,ce dernier réagissant avec la fonction carboxylique d'un acide aminé dont la fonction amine est protégée par le groupement tertio-butyloxycarbonyle, le groupement protecteur de la fonction amine étant éliminé à la fin, le stéroïde pouvant être l'hydrocortisone, ou la dexaméthasone ou tout autre stéroïde glucocorticoïde à chaîne-CO-CH₂OH, l'acide aminé étant en particulier la cystine, la méthionine, ou la cystéine dont la fonction thiol est protégée.

3. Procédé de préparation des dérivés de formule II, selon la revendication 1, obtenus par transformations successives du stéroïde en mésylate puis en dérivé iodé, ce dernier réagissant avec la fonction amine d'un acide aminé dont la fonction carboxylique est protégée par différents groupements dont les esters, la réaction pouvant être réalisée dans les mêmes conditions à partir du mésylate - 21 du stéroïde, le stéroïde pouvant être l'hydrocortisone, ou la dexaméthasone ou tout autre stéroïde, glucocorticoïde à chaîne-CO-CH₂OH, l'acide aminé pouvant être en particulier la cystine, la méthionine, l'acide glutamique,ou encore l'acide pyroglutamique.

4. Procédé de préparation des dérivés de formule III, selon la revendication 1, obtenue par transformation du stéroïde en mésylate qui réagit avec un acide aminé présentant un groupement thiol SH libre, tel que par exemple, la cystéine, ou l'homocystéine.

5. Composés de formules I, II ou III selon la revendication 1, obtenus d'après le procédé de préparation selon l'une quelconque des revendications 2 à 4, caractérisés en ce qu'ils représentent avantageusement l'un des composés ci-après:
- ester entre l'hydrocortisone et la cystine (formule I);
- ester entre la dexaméthasone et la cystine (formule I);
- ester entre l'hydrocortisone et la méthionine (formule I);
- ester entre la dexaméthasone et le méthionine (formule I);
- amine en C₂₁ entre l'hydrocortisone et la méthionine (formule II);
- amine en C₂₁ entre la dexaméthasone et la méthionine (formule II);
- amine en C₂₁ entre l'hydrocortisone et la cystine (formule II);
- amine en C₂₁ entre la dexaméthasone et la cystine (formule II);
- éther thiol en 21 entre l'hydrocortisone et la cystéine (formule III);
- éther thiol en 21 entre la dexaméthasone et la cystéine (formule III);

6. Composés de formules I, II ou III, selon l'une quelconque des revendications 1 et 5, obtenus d'après le procédé de préparation selon l'une quelconque des revendications 2 à 4, sous forme de sels d'addition avec les acides minéraux ou organiques en présence du groupement aminé, ou leurs sels minéraux en présence du groupement carboxylique.

7. Composés selon la revendication 6, sous forme de sels d'amines avec les acides minéraux ou organiques, ou sous forme de carboxylates de sodium de potassium, ou de calcium.

8. Application des composés selon l'une quelconque des revendications 1, 5 à 7 pour la fabrication d' un médicament destiné à une utilisation thérapeutique comme anti-inflammatoire.

9. Application des composés selon la revendication 8 pour la fabrication d' un médicament destiné à une utilisation thérapeutique comme anti-inflammatoire, particulièrement au niveau cutané et ophtalmique.

## Claims

1. Compounds, characterised in that they are associated synthesis compounds of sulphurized or non-sulphurized amino acids with derivatives of Δ-4 pregnane, 3,20-dione or with derivatives of Δ-1,4 pregnadiene 3,20-dione, and in that they correspond to the three general formulae I, II and III: 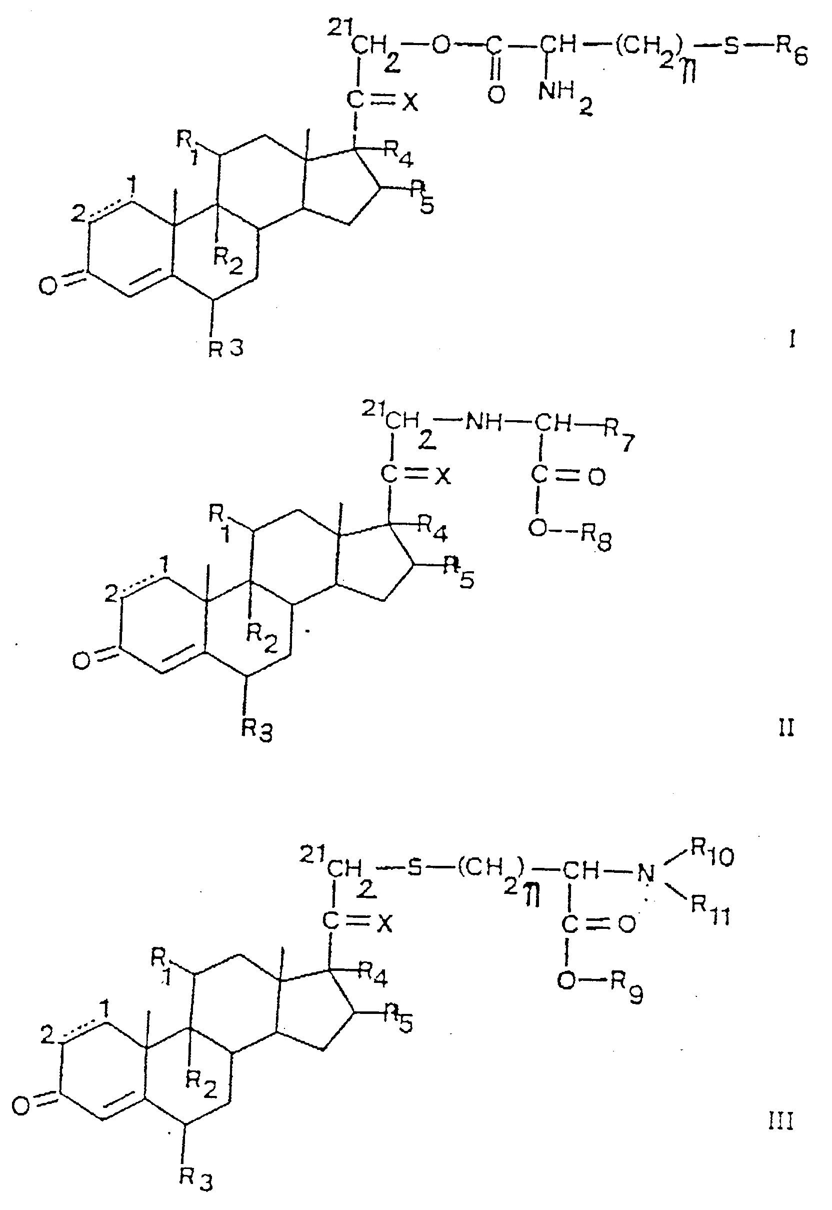 in which:
in the three general formulae I, II and III,
the C1-C2 bond may be a saturated bond (for example hydrocortisone derivatives) or may be an ethylene bond (for example dexamethasone derivatives);
R1 represents an oxygen, sulphur or chlorine atom or again a hydroxyl;
R2 and/or R3 represent a hydrogen, fluorine or chlorine atom or again a methyl grouping;
R4 and/or R5 represent a hydrogen or a hydroxyl or a methyl or an ethyl;
R5 may also be a methylene;
R4 and R5 may form a heterocycle of the -O-CH(Y)-O- or -O-C(Y)2-O- type, Y being a hydrogen or a linear or branched alkyl group preferably having 1 to 4 carbon atoms;
X represents an oxygen or a sulphur;
- in formula I
n is 1 or 2;
R6 may represent a methyl or an arylalkyl grouping or any other grouping used to protect the thiol function;
R6 may correspond to the entire structure I, duplicating formula I with formation of a disulphide bridge;
- in formula II
R7 represents a hydrogen of glycine origin or a linear alkyl group, in particular a methyl of alanine origin or a branched alkyl group of valine, leucine, isoleucine origin or a linear hydroxyalkyl group of serine, threonine origin or a thioalkyl group of methionine origin or a thioalkyl group of cysteine origin or an arylalkyl group that is for example benzyl of phenylalanine origin, or for example hydroxybenzyl of tyrosine origin, or for example indolylalkyl of tryptophane origin, or imidazolylalkyl of histidine origin or an amidoalkyl group of asparagine, glutamine origin or a carboxylic alkyl group in a free form or salified by sodium, for example of aspartic acid, glutamic acid origin or aromatised in the form of pyroglutamic acid or an alkyl amino group of lysine origin or a guanidinoalkyl group of arginine origin or again a dithiodimethyl group of cystine origin symmetrically fixing two glucocorticoids;
the NH grouping may form, with R7, a nitrogenous heterocycle of the pyrrolidine type of proline origin;
the various amino acids mentioned may be replaced by their higher homologues;
R8 may be a hydrogen or a corresponding salt of the sodium, potassium or calcium type;
R8 may also be a linear or branched alkyl group preferably containing 1 to 4 carbon atoms or an arylalkyl group of benzyl or heterocyclic type;
- in formula III
n is 1 to 6, but it is preferable if n = 1 of cysteine origin or n = 2 of homocysteine origin;
R9 may assume the various structures mentioned for R8;
R10 and/or R11 may represent a hydrogen or a linear or branched alkyl grouping preferably containing 1 to 3 carbon atoms, a structure in amino form or ammonium;
R10 or R11 may also have a -CO-Z- structure, Z being a linear or branched alkyl or arylalkyl grouping.

2. Process for preparing derivatives corresponding to formula I according to claim 1 obtained by successive transformation of the steroid into mesylate then into an iodated derivative, the iodated derivative reacting with the carboxylic function of an amino acid of which the amine function is protected by the tertio-butyloxycarbonyl grouping, the protective grouping of the amine function eventually being eliminated, wherein the steroid may be hydrocortisone or dexamethasone or any other glucocorticoid steroid with a -C0-CH2OH chain, wherein the amino acid is, in particular, cystine, methionine, or again cysteine of which the thiol function is protected.

3. Process for preparing derivatives corresponding to formula II according to claim 1 obtained by successive transformation of the steroid into mesylate then into an iodated derivative, the iodated derivative reacting with the amine function of an amino acid of which the carboxylic function is protected by various groupings including esters, wherein the reaction may be carried out under the same conditions from the 21-mesylate of the steroid, wherein the steroid may be hydrocortizone or dexamethasone or any other glucocorticoid steroid with a -C0-CH2OH chain, wherein the amino acid may be, in particular, cystine, methionine, glutamic acid or again a pyroglutamic acid.

4. Process for preparing derivatives corresponding to formula III according to claim 1 obtained by transformation of the steroid into mesylate which reacts with an amino acid containing a free SH thiol grouping such as, for example, cysteine or homocysteine.

5. Compounds corresponding to formulae I, II, or III, according to claim 1 obtained by the preparation process according to any one of claims 2 to 4, characterised in that they advantageously represent one of the following compounds:
- ester between hydrocortisone and cystine (formula I);
- ester between dexamethasone and cystine (formula I);
- ester between hydrocortisone and methionine (formula I);
- ester between dexamethasone and methionine (formula I);
- C21 amine between hydrocortisone and methionine (formula II);
- C21 amine between dexamethasone and methionine (formula II);
- C21 amine between hydrocortisone and cystine (formula II);
- C21 amine between dexamethasone and cystine (formula II);
- 21 thiol ether between hydrocortisone and cysteine (formula III);
- 21 thiol ether between dexamethasone and cystine (formula III).

6. Compounds corresponding to formulae I, II or III, according to any one of claims 1 and 5 obtained by the preparation process according to any one of claims 2 to 4 in the form of addition salts with mineral or organic acids in the presence of the amino grouping or mineral salts thereof in the presence of the carboxylic grouping.

7. Compounds according to claim 6 in the form of amine salts with mineral or organic acids or in the form of sodium, potassium or calcium carboxylates.

8. Application of the compounds according to any one of claims 1, 5 to 7, for the production of a drug intended for therapeutic use as an anti-inflammatory agent.

9. Application of the compounds according to claim 8 for the production of a drug intended for therapeutic use as an anti-inflammatory agent, in particular in the cutaneous and ophthalmic region.

## Patentansprüche

1. Zubereitungen, gekennzeichnet dadurch, daß sie mittels Synthese miteinander vereinigte schwefelhaltige oder nicht-schwefelhaltige Aminosäuren mit Derivaten von Δ -4 Pregnen-3,20-dion oder mit Derivaten von Δ -1,4 Pregnadien-3,20-dion sind, und daß sie den drei allgemeinen Formeln I , II und III entsprechen: 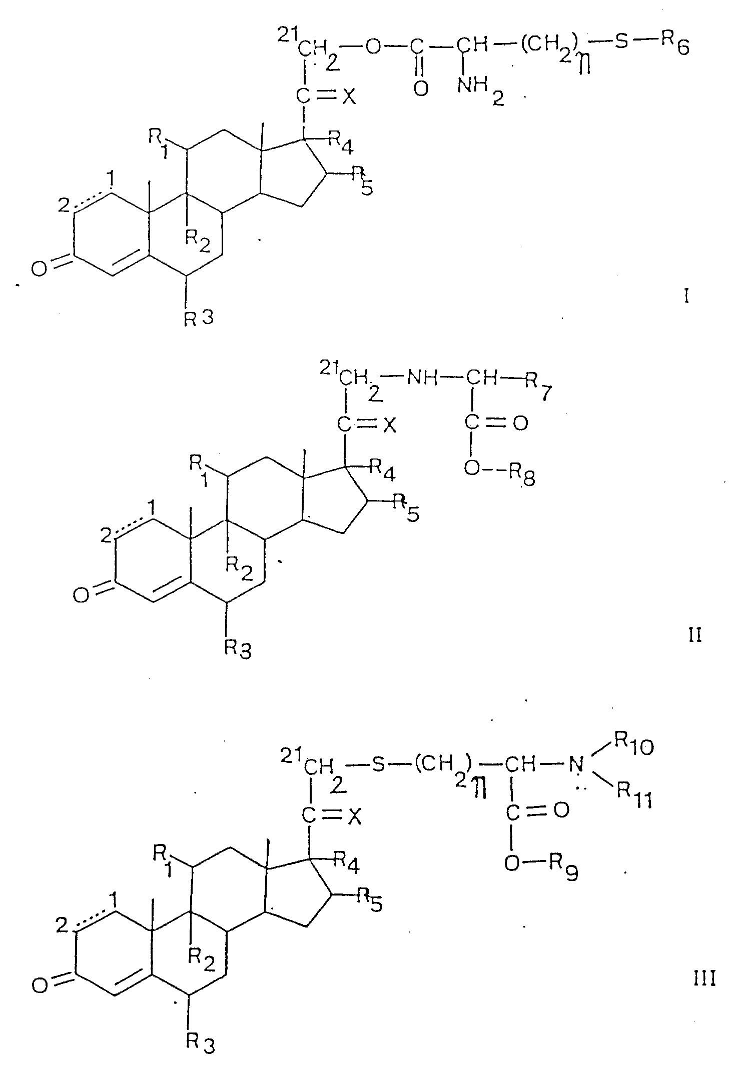 worin:
bei den drei allgemeinen Formeln I , II und III die Bindung C₁-C₂ gegebenenfalls eine gesättigte Bindung ist, (z.B. von Hydrocortison abgeleitet) oder gegebenenfalls eine ethylenische Bindung (z.B. abgeleitet von Dexamethason) ist,
R₁ ein Sauerstoff- oder Schwefel- oder Chloratom oder auch eine Hydroxylgruppe darstellt,
R₂ und/oder R₃ ein Wasserstoffatom oder ein Fluoratom oder ein Chloratom oder auch eine Methylgruppe darstellen
R₄ und/oder R₅ ein Wasserstoffatom, eine Hydroxylgruppe oder eine Methylgruppe oder eine Ethylgruppe darstellen, R₅ außerdem eine Methylengruppe sein kann,
R₄ und R₅ eine heterocyclische Gruppe des Typs O-CH-(Y)-0- oder -0-C-(Y)₂-0 bilden können,
Y ein Wasserstoffatom oder eine lineare oder verzweigte Alkylgruppe vorzugsweise mit bis zu 4 Kohlenstoffatomen ist,
X ein Sauerstoff- oder ein Schwefelatom darstellt und daß in Formel I
n 1 oder 2 ist,
R₆ eine Methyl oder eine Arylalkylgruppe darstellt oder eine andere Gruppe sein kann, die zum Schutz einer Thiolfunktion gebraucht wird,
R₆ gegebenenfalls auch einer Gruppierung der Struktur I entsprechen kann, die eine Verdoppelung der Formel I unter Bildung einer Disulfidgruppe sein kann,
und wobei in Formel II
R₇ ein Wasserstoffatom mit dem Ursprung von Glycin oder eine lineare Alkylgruppe, insbesondere eine. Methylgruppe mit dem Ursprung von Alanin darstellt oder eine verzweigte Alkylgruppe mit dem Ursprung von Valin, Leucin, Isoleucin oder eine lineare Hydroxyalkylgruppe sein kann, die von Serin, Threonin stammt, oder eine Thioalkylgruppe, die von Methionin stammt, oder eine Thioalkylgruppe, die von Cystein stammt, oder eine Arylalkylgruppe, die z.B. eine Benzylgruppe sein kann, die von Phenylalanin abstammt, bzw. z.B. eine Hydroxybenzylgruppe, die von Tyrosin abstammt, oder z.B. eine Indolylalkylgruppe, die von Tryptophan abstammt, oder eine Imidezolylalkylgruppe, die von Histidin abstammt, oder eine Aminoalkylgruppe, die von Asparagin, Glutamin abstammt, oder eine Carboxylalkylgruppe ist, die in freier Form oder als Salz z.B. als Natriumsalz vorliegt und von Asparaginsäure, Glutaminsäure oder cyclisiert unter Bildung einer Pyroglutaminsäure abstammt, oder eine Aminoalkylgruppe ist, die von Lysin abstammt, oder eine Guanidinoalkyl, die von Arginin abstammt, oder auch eine Dithiodimethylgruppe, die von Cystin abstammt, darstellt und symmetrisch 2 Glucocorticoide verbindet, wobei die Gruppe NH auch mit R₇ eine Stickstoff-heterocyclische Verbindung des Typs Pyrrolidin bilden kann, die von Prolin abstammt, wobei die verschiedenen genannten Aminosäuren durch ihre höheren Homologen ersetzt sein können,
R₈ ein Wasserstoffatom oder ein Salz entsprechend dem Typ Natrium, Kalium oder Calcium sein kann,
R₈ auch eine lineare oder verzweigte Alkylgruppe mit vorzugsweise 1-4 Kohlenstoffatomen sein kann oder eine Arylalkylgruppe des Typs Benzyl oder eine heterocyclische Gruppe sein kann und wobei
in der Formel III
n 1-6 sein kann, aber vorzugsweise n = 1 mit dem Ursprung Cystein oder n = 2 mit dem Ursprung Homocycstein ist,
R₉ eine unterschiedliche Struktur, wie sie durch R₈ dargestellt ist, sein kann,
R₁₀ und/oder R₁₁ ein Wasserstoffatom oder eine lineare oder verzweigte Alkylgruppe vorzugsweise mit 1 - 3 Kohlenstoffatomen darstellen kann, wobei diese Struktur aminiert oder mittels Ammonium gebildet sein kann,
R₁₀ oder R₁₁ auch eine Struktur -CO-Z- haben kann, wobei Z eine lineare oder verzweigte Alkylgruppe oder eine Arylalkylgruppe ist.

2. Verfahren zur Herstellung von Derivaten der Formel I nach Anspruch 1 , erhalten durch nacheinander erfolgende Überführung des Steriods in das Mesylat und anschließend in das jodierte Derivat davon und man das letztere mit der Carboxylfunktion einer Aminosäure umsetzt, deren Aminfunktion durch eine tertiäre Butyloxycarbonylgruppe geschützt ist, wobei die Schutzgruppe der Aminfunktion schließlich abgetrennt wird und das Steroid, das Hydrocortison sein kann oder Dexamethason oder ein anderes glucocorticoides Steroid mit einer Kette -CO-CH₂OH sein kann, wobei die Aminosäure insbesondere Cycstin, Methionin oder Cystein sein kann, deren Thiolgruppe geschützt ist.

3. Verfahren zur Herstellung von Derivaten der Formel II nach Anspruch 1 , erhalten durch nacheinander erfolgende Umsetzungen des Steroids in das Mesylat und weiter in dessen jodiertes Derivat, wobei das letztere mit der Aminfunktion einer Aminosäure umgesetzt wird, deren Carboxylfunktion durch verschiedene Gruppen geschützt ist, wie durch Ester, wobei die Reaktion durchgeführt werden kann unter den gleichen Bedingungen über das Mesylat-21 des Steroids, wobei das Steroid Hydrocortison oder Dexamethason oder ein anderes glucocorticoides Steroid sein kann, dessen Kette -CO-CH₂OH ist, wobei die Aminosäure insbesondere Cystin, das Methionin, die Glutaminsäure oder auch Pyroglutaminsäure sein kann.

4. Verfahren zur Herstellung von Derivaten der Formel III nach Anspruch 1, erhalten durch Umsetzung des Steroids in das Mesylat, das mit einer Aminosäure reagiert, die eine freie Thiolgruppe SH aufweist, wie z.B. das Cystein oder Homocycstein.

5. Zubereitungen der Formeln I , II oder III nach Anspruch 1, erhalten nach dem Herstellungsverfahren nach einem der Ansprüche 2 bis 4, dadurch gekennzeichnet, daß sie vorzugsweise eine der folgenden Zusammensetzungen darstellen:
Ester zwischen Hydrocortison und Cystin (Formel I),
Ester zwischen Dexamethason und Cystin (Formel I),
Ester zwischen Hydrocortison und Methionin (Formel I),
Ester zwischen Dexamethason und Methionin (Formel I),
Amin in C₂₁ zwischen Hydrocortison und Methionin (Formel II),
Amin in C₂₁ zwischen Dexamethason und Methionin (Formel II),
Amin in C₂₁ zwischen Hydrocortison und Cystin (Formel II),
Amin in C₂₁ zwischen Dexamethason und Cystin (Formel II),
Etherthiol in 21 zwischen Hydrocortison und Cystein (Formel III),
Etherthiol in 21 zwischen Dexamethason und Cystein (Formel III),

6. Zubereitungen der Formeln I , II oder III nach einem der Ansprüche 1 und 5, erhalten nach dem Herstellungsverfahren nach einem der Ansprüche 2 bis 4 unter Bildung von Additionssalzen mit Mineralsäuren oder organischen Säuren in Gegenwart einer aminierten Gruppe oder deren Mineralsalze, in Gegenwart einer Carboxlgruppe.

7. Zusammensetzung nach Anspruch 6 unter Bildung von Aminsalzen mit Mineralsäuren oder organischen Säuren oder unter Bildung von Carboxylaten des Natriums, des Kaliums oder des Calciums.

8. Anwendung von Zubereitungen nach einem der Ansprüche 5 bis 7 durch Erzeugung eines Medikaments, bestimmt zur therapeutischen Anwendung als entzündungshemmendes Mittel.

9. Anwendung von Zubereitungen nach Anspruch 8 zur Erzeugung eines Medikaments, das durch therapeutischen Gebrauch als Anti-entzündungsmittel, insbesondere auf dem Gebiet der Haut oder der Augenheilkunde bestimmt ist.
